# EUROPEAN PATENT SPECIFICATION

(11) **EP 3 030 145 B1**
(45) Date of publication and mention of the grant of the patent: **30.05.2018**
(21) Application number: 14744327.9
(22) Date of filing: 28.07.2014
(51) Int. Cl.: A61B 5/024, A61B 5/021

(54) **MONITORING SYSTEM AND METHOD FOR MONITORING THE HEMODYNAMIC STATUS OF A SUBJECT**
ÜBERWACHUNGSSYSTEM UND VERFAHREN ZUR ÜBERWACHUNG DES HÄMODYNAMISCHEN ZUSTANDS EINER PERSON
SYSTÈME ET PROCÉDÉ DE SURVEILLANCE DE L'ÉTAT HÉMODYNAMIQUE D'UN SUJET

(30) Priority: 07.08.2013 US 201361863017 P; 07.08.2013 EP 13179554
(43) Date of publication of application: 15.06.2016
(73) Proprietor: Koninklijke Philips N.V., 5656 AE Eindhoven (NL)
(72) Inventor: MÜHLSTEFF, Jens, NL-5656 AE Eindhoven (NL); KIRENKO, Ihor Olehovych, NL-5656 AE Eindhoven (NL)
(74) Representative: Ledeboer, Johannes Albertus
(86) International application number: PCT/EP2014/066118
(87) International publication number: WO 2015/018675

(56) References cited:
- WO-A1-2013/093690
- DE-A1-102011 016 772
- VIKRAM CHANDRASEKARAN ET AL: "Cuffless Differential Blood Pressure Estimation Using Smart Phones", IEEE TRANSACTIONS ON BIOMEDICAL ENGINEERING, IEEE SERVICE CENTER, PISCATAWAY, NJ, USA, vol. 60, no. 4, 1 April 2013 (2013-04-01), pages 1080-1089, XP011497327, ISSN: 0018-9294, DOI: 10.1109/TBME.2012.2211078
- WONG M Y M ET AL: "Contactless recording of photoplethysmogram on a sleeping bed", PROCEEDINGS OF THE 31ST ANNUAL INTERNATIONAL CONFERENCE OF THE IEEE ENGINEERING IN MEDICINE AND BIOLOGY SOCIETY: ENGINEERING THE FUTURE OF BIOMEDICINE, EMBC 2009, IEEE, 3 September 2009 (2009-09-03), pages 907-910, XP031882404, DOI: 10.1109/IEMBS.2009.5334560 ISBN: 978-1-4244-3296-7 cited in the application
- .: "INNOVATION CONVERSATION: What Some Xerox Researchers Do on their Lunch Hour ...", , 8 February 2013 (2013-02-08), pages 1-1, XP055066382, Retrieved from the Internet: URL:http://news.xerox.com/news/Xerox-featu re-story-innovation-conversation-with-rese archers-on-lunch-hour [retrieved on 2013-06-12]

## Description

### FIELD OF THE INVENTION

The present invention relates to monitoring system and a corresponding method for monitoring the hemodynamic status of a subject, in particular for monitoring a change in blood pressure of a subject, such as a patient or a premature baby.

### BACKGROUND OF THE INVENTION

In the field of medical monitoring systems monitoring devices are available having an accelerometer disposed at the apex position in order to detect the pulse and the respiration of a patient and a cuff-based blood pressure monitor at the upper arm and, further, a photo-plethysmography (PPG) sensor at the wrist or the fingertip. These sensors allow continuous monitoring of a patient.

Early recognition of subtle signs of patient deterioration has been found as a major problem to be addressed by patient monitoring systems.

A continuous blood pressure measurement may be necessary for certain patients having e.g. hemodynamic instability which may lead to an increased risk for the patient. Further, this is often of importance for monitoring premature babies in a neonatal intensive care unit (NICU). Non-invasive blood pressure measurements are mainly based on the sphygmo-manometric occlusive arm-cuff, which allows only intermittent measurements. This method is not only uncomfortable for the patient, or even difficult to use e.g. for premature babies, but it is also possible that critical blood pressure changes can be missed during time-intervals without a blood pressure measurement. Blood pressure is typically measured only once or twice a day in low acuity settings.

Therefore there is a strong interest for monitoring devices that provide continuous detection of significant changes of the hemodynamic status and in particular the blood pressure.

A method to monitor blood pressure changes known from the state of the art is based on the pulse arrival time methodology, wherein the heart rate is acquired from an electrocardiogram and the blood pressure changes are determined in combination with a photoplethysmogram, which is acquired by a respective photo-optical sensor at the fingertip, the ear, forehead or the wrist of the patient. The pulse arrival time is determined as the sum of the pre-ejection period and the pulse transit time, wherein the pre-ejection period, the period of iso-volumetric contraction, can vary independently of the blood pressure so that the reliability of this method is reduced. However using the relative changes of the pulse arrival time has been demonstrated as a feasible parameter for early detection of impending faints due to blood pressure reduction, wherein a decrease of 20 mm Hg is associated with 20 ... 40 milliseconds change in the pulse arrival time.

WO 2013/093690 A1 discloses an apparatus for use in monitoring the baroreceptor reflex in a user. The apparatus comprises a processor configured to process a signal output by a first sensor that is attached to or located proximate to a bed to determine when the user moves from a lying position on the bed to a sitting position, and to provide an indication of the baroreceptor reflex of the user by processing the signal to determine the change in the heart rate of the user that occurs as a result of moving from the lying position to the sitting position.

Wong M. Y. M. et al.: "Contactless recording of photoplethysmogram on a sleeping bed", PROCEEDINGS OF THE 31ST ANNUAL INTERNATIONAL CONFERENCE OF THE IEEE ENGINEERING IN MEDICINE AND BIOLOGY SOCIETY: ENGINEERING THE FUTURE OF BIOMEDICINE, EMBC 2009, IEEE, 3 September 2009 (2009-09-03), pages 907-910, discloses a contactless monitoring method to record reflective mode photoplethysmogram (PPG) on a sleeping bed for heart rate (HR) estimation. The electrocardiogram (ECG) and pulse transit time (PTT) were also measured in this study. ECG was measured from subjects' limbs whilst PPG was obtained from their right index fingers and their backs with and without direct contact between the PPG sensor and the subjects' skin respectively. Clear PPG waveforms were obtained from the subjects' backs even though the sensor was not directly attached to their skin. Beat-to-beat HRs derived from the back PPGs were closely correlated with those measured from the finger PPGs and ECGs.

VIKRAM CHANDRASEKARAN ET AL: "Cuff!ess Differential Blood Pressure Estimation Using Smart Phones", IEEE TRANSACTIONS 0N BIOMEDICAL ENGINEERING, IEEE SERVICE CENTER, PISCATAWAY, NJ, USA, vol. 60, no. 4, 1 April 2013, pages 1080-1089, discloses two differential blood pressure estimating techniques using the heartbeat and pulse data. The first method use.s two smart phones whereas the second method replaces one of the phones with a customized external microphone. The systolic and diastolic pressure in the two techniques are estimated by computing the pulse pressure and the stroke volume from the data recorded.

A monitoring system for monitoring the hemodynamic status, in particular for the detection of a hemodynamic crisis, of a subject, e.g. a premature baby in neonatal care, without on-body sensors (e.g. electrodes that harm the skin of neonates) is currently not available.

### SUMMARY OF THE INVENTION

It is an object of the present invention to provide an improved monitoring system and method for monitoring the hemodynamic status of a subject that enables a continuous monitoring with low technical effort, an increased comfort and improved safety for the subject and that is particularly usable for monitoring of premature babies.

According to the present invention, a monitoring system for monitoring the hemodynamic status of a subject is presented, said monitoring system comprising:
- an imaging unit configured to obtain a plurality of images of the subject from a remote distance over time,
- a sensor unit configured to acquire a sensor signal of the subject related to a vital sign of the subject,
- a PPG unit configured to generate photo-plethysmography, PPG, signals from various body sites of the subject from said plurality of images, and
- an evaluation unit configured
   to commonly evaluate said acquired sensor signal and said PPG signals,
   to extract pulse arrival time, PAT, measures from said sensor signal and said PPG signals, and
   to extract a hemodynamic information about the hemodynamic status of the subject (100) from said PAT measures. Further, according to the present invention, there is provided a corresponding method.

Preferred embodiments of the invention are defined in the dependent claims. It shall be understood that the claimed methodhas similar and/or identical preferred embodiments as the claimed system and as defined in the dependent claims.

The present invention is based on the idea to exploit a contactless method for obtaining a PPG signal that is used as one element in the extraction of hemodynamic information (e.g. the blood pressure) about the hemodynamic status of the subject. Said contactless method is generally known and uses the principle of remote PPG for obtaining vital signs, such as heart rate, breathing rate or SpO2, of a subject. It uses an imaging unit, such as a camera (e.g. a video camera), to a time series of image frames of the subject, which is then used for generating a PPG signal in a known manner.

The present invention uses as a second element a sensor signal that is related to a vital sign of the subject and that is acquired by a sensor unit (including one or more identical or different sensors). This sensor unit is preferably configured for measurement of the sensor signal without arranging sensors to the body of the subject as well.

By way of a combined analysis of the PPG signal and the sensor signal reliable and immediate information about the hemodynamic status and particularly hemodynamic status changes of the subject can be obtained without using sensing means attached to the subject's body, preferably in a contactless manner. Further, a continuous monitoring is easily possible. The proposed system thus provides an increased comfort and can be used for continuous monitoring of premature babies, e.g. in an NICU.

According to an embodiment said evaluation unit is configured to determine the change in the blood pressure from the common evaluation of said sensor signal and said PPG signal. The PPG signal delivers information about the blood volume pulse and the sensor signal delivers information about a vital sign, such as the heart rate, or even represents an ECG signal. From the combination of these two signals, a certain time duration signal may be determined which corresponds to the speed with which the cardiac-induced pressure wave travels in the subject's arterial system. A change in blood pressure may then be determined on the basis of this time duration signal. Since the blood pressure is merely determined by measuring the PPG signal and the sensor signal, the technical effort for determining changes in blood pressure is reduced, the subject does not need to wear a (e.g. cuff-based) blood pressure measurement device and trends in blood pressure can be measured continuously and nearly in real-time so that a high reliability of this measurement is ensured.

According to the present invention said evaluation unit is configured to extract pulse arrival time, PAT, measures and to extract hemodynamic information about the hemodynamic status of the subject from said PAT measures. Non-limiting examples of such PAT measures are the PAT foot, PAT 20%, PAT50 %, PAT80 %, the PAT top, the pulse transit time and/or the pre-ejection period. Such PAT measures are generally known from the known PAT methodology. From a combination of the PPG signal and the sensor signal such PAT measures can be easily obtained and monitored to detect changes of the hemodynamic status of the subject. Measuring a PPG signal from various body sites, e.g. from the forehead and foot, allows to detect Pulse Transit Time measures (PTT), which are not affected by the pre-ejection period. More precisely, in mathematical terms, taking the difference of at least two measured PAT cancels the PEP contribution and only the PTT difference remains.

Preferably, said evaluation unit is configured to determine a start signal of a PAT measure from the sensor signal and an end signal of the PAT measure from a systolic measurement value of the PPG signal. A start time and an end time can thus be determined with low technical effort and high precision.

According to another embodiment said evaluation unit is configured to detect changes in one or more PAT measures and to issue an indication signal indicating if changes in one or more PAT measures have been detected. For instance, an emergency or alarm can be signalled to a caregiver, nurse or physician, e.g. via a pager, phone or other signalling means, in case a serious change of the hemodynamic change has been detected.

Said evaluation unit is then advantageously configured to compare one or more extracted PAT measures to reference PAT measures previously acquired for the same subject or for a different subject or formed from PAT measures acquired for a plurality of subject (e.g. formed as an average of PAT measures from different subjects). This further increases reliability and accuracy of the monitoring of the hemodynamic status.

The sensor unit preferably comprises an ECG sensor, in particular a capacitive ECG sensor, for acquiring an ECG sensor signal allowing the extraction of a heart rate signal related to the heart rate of the subject. Further, in an embodiment sensor unit, additionally or alternatively, comprises a pressure sensor for acquiring a pressure sensor signal representing pressure changes allowing the extraction of a heart rate signal related to the heart rate of the subject and/or a respiration signal related to the respiration rate of the subject. Such an ECG sensor and such a pressure sensor are generally known and can e.g. be embedded into the mattress or a textile of a patient support, e.g. a patient bed.

Respiration affects PAT measures, since the intra-thoracic pressures are modulated by respiration effort. The analysis of the correlation of respiration effort and PAT measures allows determining e.g. apnea events during sleep. PPG pulse signal width provides information on the systemic vascular resistance and is used to infer centralization or vasodilatation processes e.g. during critical events such as cardiac arrest.

In case the sensor unit comprises both an ECG sensor and a pressure sensor the evaluation unit is configured to extract pulse arrival time, PAT, measures from different combinations of PPG signal, ECG sensor signal and pressure sensor signal, to check said PAT measures for consistency and to use the result of said check in the extraction of a hemodynamic information about the hemodynamic status of the subject. In this way the accuracy and reliability of the monitoring result can be further increased. Inconsistent results may e.g. be ignored so that only consistent results are used for determining the hemodynamic changes. If all PAT measures obtained from different combinations of signals are inconsistent this may be interpreted in an embodiment as an indication of low signal quality or inaccurate measurement of one or more signals, in which case a corresponding output signal maybe issued so that the user can check the measurement setup.

In still another embodiment the monitoring system may further comprise a blood pressure measurement unit, e.g. a cuff-based blood pressure measurement device, for measuring the blood pressure of the subject. This blood pressure measurement unit may be used for intermittent measurement to verify the determined hemodynamic information, e.g. change in blood pressure, by means of a precise measurement from time to time and to calibrate PAT measurements.

In a further preferred embodiment, the evaluation unit is configured to control a blood pressure measurement unit on the basis of the determined hemodynamic information. Hence, the blood pressure measurement may only be triggered if a significant change of the hemodynamic status is detected to reduce the frequency of the blood pressure measurements.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an embodiment of a monitoring system according to the present invention,
Fig. 2 shows a diagram of an electrocardiogram and a photo-plethysmogram for illustration of the PAT methodology,
Fig. 3 shows a diagram illustrating the typical PAT increase in case of a blood pressure decrease,
Fig. 4 shows a flow chart illustrating a first embodiment of a monitoring method according to the present invention,
Fig. 5 shows a flow chart illustrating a second embodiment of a monitoring method according to the present invention, and
Fig. 6 shows a flow chart illustrating a third embodiment of a monitoring method according to the present invention.

### DETAILED DESCRIPTION OF THE INVENTION

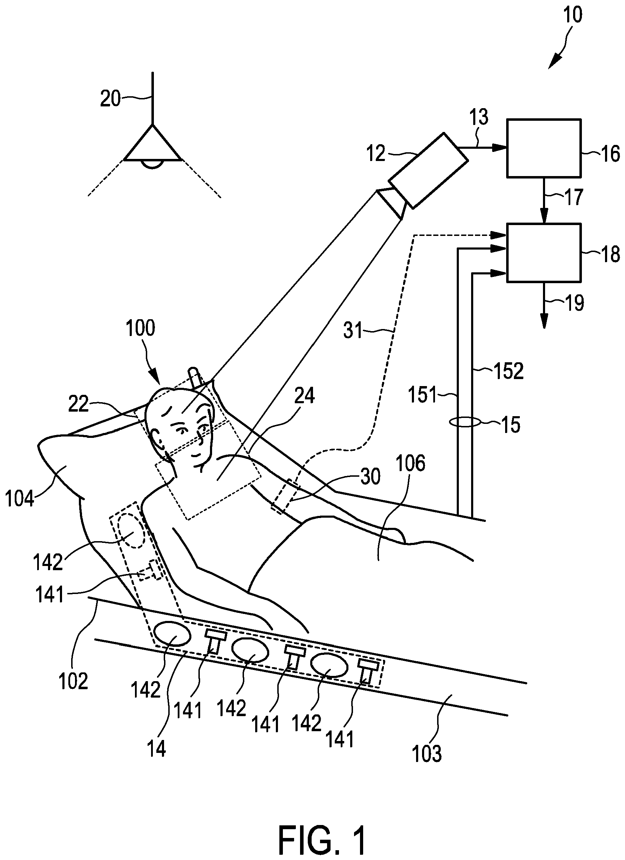

Fig. 1 shows a schematic diagram of a monitoring system 10 according to the present invention for monitoring the hemodynamic status of a subject 100, e.g. a patient, lying in a bed 102, wherein the head of the subject 100 is located on a pillow 104 and the subject 100 is covered with a blanket 106. The system 10 comprises an imaging unit 12 for obtaining a plurality of images 13 of the subject 100 over time, a sensor unit 14 for acquiring a sensor signal 15 of the subject 100 related to a vital sign of the subject 100, a PPG unit 16 for generating a photo-plethysmography, PPG, signal 17 from said plurality of images, and an evaluation unit 18 for commonly evaluating said sensor signal 15 and said PPG signal 17 to extract a hemodynamic information 19 about the hemodynamic status of the subject 100.

In this setup the imaging unit 12 is installed at a remote distance, for example at a ceiling or a wall of a room in which the bed 102 is located. Generally, ambient light is sufficient for illuminating the scene, but an optional light source 20 can be present to illuminate the scenery and to ensure sufficient image contrast. In one embodiment, the imaging unit 20 can be a monochrome or single color camera, e.g. an infrared camera or a video camera, and the light source 20 can be a corresponding light source, e.g. an infrared light source. It is to be understood, that in further embodiments the imaging unit 12 can be adapted to detect light in the visible and/or infrared spectral range and the light source 20 can be adapted to emit light in the infrared and/or visible spectral range. In this embodiment, the subject 100 and the imaging unit 12 are located oppositely to one another, but it is to be understood that the imaging unit 12 and the light source 20 can in principle be arbitrarily oriented with respect to the subject 100.

In the PPG unit 16, which may be implemented as software running on a processor or computer and/or as dedicated hardware, a PPG signal 17 is generated from the plurality of images 13 acquired by the imaging unit. Plethysmography generally refers to the measurement of volume changes of an organ or a body part and in particular to the detection of volume changes due to a cardio-vascular pulse wave traveling through the body of a subject with every heart beat. Photo-plethysmography (PPG) is an optical measurement technique that evaluates a time-variant change of light reflectance or transmission of an area or volume of interest. PPG is based on the principle that blood absorbs light more than surrounding tissue, so variations in blood volume with every heart beat affect transmission or reflectance correspondingly. Besides information about the heart rate, a PPG waveform can comprise information attributable to further physiological phenomena such as the respiration. By evaluating the transmissivity and/or reflectivity at different wavelengths (typically red and infrared), the blood oxygen saturation can be determined.

Conventional pulse oximeters for measuring the heart rate and the (arterial) blood oxygen saturation of a subject are attached to the skin of the subject, for instance to a finger tip, earlobe or forehead. Therefore, they are referred to as 'contact' PPG devices. A typical pulse oximeter comprises a red LED and an infrared LED as light sources and one photodiode for detecting light that has been transmitted through patient tissue. Commercially available pulse oximeters quickly switch between measurements at a red and an infrared wavelength and thereby measure the transmissivity of the same area or volume of tissue at two different wavelengths. This is referred to as time-division-multiplexing. The transmissivity over time at each wavelength gives the PPG waveforms for red and infrared wavelengths. Although contact PPG is regarded as a basically non-invasive technique, contact PPG measurement is often experienced as being unpleasant, since the pulse oximeter is directly attached to the subject and any cables limit the freedom to move.

According to the present invention the meanwhile introduced non-contact remote PPG technique for unobtrusive measurements is applied according to which a time series of images of one or more skin portions of the subject are evaluated as e.g. described by Verkruysse et al., "Remote plethysmographic imaging using ambient light", Optics Express, 16(26), 22 December 2008, pp. 21434-21445, who demonstrates that PPG signals can be measured remotely using ambient light and a conventional consumer level video camera. Subtle color changes of the skin caused by blood volume pulses are thus detected. The region of interest 22 is typically the face, in particular the forehead or the cheeks, but there may be other uncovered parts of skin areas (e.g. the neck or throat) on the subject, for instance the body of a neonate that only wears a diaper. The same camera can also measure respiration rates by monitoring breathing motion of the chest and/or belly of the subject using a different region of interest 24.

The sensor unit 14 for acquiring a sensor signal 15 of the subject 100 related to a vital sign (e.g. heart rate or respiration rate) of the subject 100 preferably comprises one or more pressure sensors 141 and/or one or more capacitive sensors 142, which are preferably arranged in the mattress 103 of the bed 102. Pressure sensors 141 (e.g. based on a piezoelectric foil) produce pressure sensor signals 151 that detect weight distributions and dynamic pressure changes to detect posture and to extract breathing movements and heart beats, e.g. based on ballistocardiography that measures ballistic forces on the heart. Capacitive sensors 142 produce (ECG like) ECG sensor signals 152 that can be used to extract heat rate information. It is to be understood that single or all sensors of the sensor unit 14 can also be integrated into a textile structure such as the blanket 106 or the pillow 104 or can be integrated into the textiles worn by the subject 100. Such sensors are e.g. disclosed in Van der Loss et. al., Unobtrusive Vital Signs Monitoring from a Multisensor Bed Sheet, RESNA'2001 Reno, NV, June 22-26, 2001 and Eilebrecht et. al., Multichannel ECG-measurement-system with capacitive patient coupling, Biomed Tech 2010, 55 (Suppl. 1), the descriptions of such sensors within these documents being incorporated herein by reference.

The evaluation unit 18, which may be implemented as software running on a processor or computer and/or as dedicated hardware, e.g. as software running on the same processor as the PPG unit 14, commonly evaluates the sensor signal 15 (including the pressure sensor signal 151 and the ECG sensor signal 152 in this embodiment) and said PPG signal 17 to extract a hemodynamic information 19 about the hemodynamic status of the subject 100.

Hemodynamic instability and blood pressure regulation failures can have severe consequences for a person. It is, for instance, associated with a higher risk to fall for adults or even critical health states such as sudden death. Falls can cause fractures, hospitalization, a longer stay in a hospital and loss of independence. Underlying root causes of such events vary a lot and might be due to structural heart diseases, dehydration, anxiety, psychological or physical stress or medication errors, which are quite common in particular for hospitalized patients.

Blood pressure measured continuously could be a parameter to detect regulation failures. However, non-invasive continuous BP measurements by the volume clamp method via conventional wearable devices are complex, heavy, prone to artifacts and require trained personal to operate.

According to a preferred embodiment of the present invention blood pressure changes or, more generally, the hemodynamic status (in particular hemodynamic changes) are monitored based on the pulse arrival time (PAT) methodology. PAT measures are derived according to an embodiment of the present invention from the PPG signal 17 and the sensor signal 15, in particular an ECG like signal derived from the pressure sensor signal 151 and/or the ECG sensor signal 152.

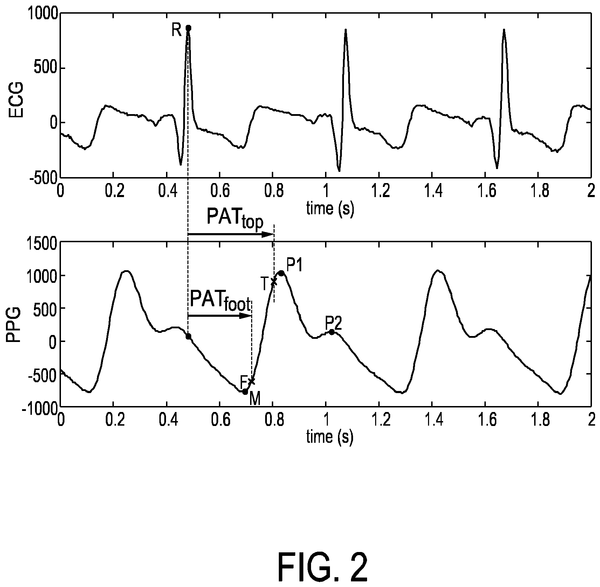

Fig. 2 shows an electrocardiogram (represented by or obtained from the sensor signal 15 according to the present invention) and a photo-plethysmogram (PPG signal 17) for evaluating the pulse arrival time. The electrocardiogram and the photo-plethysmogram are detected at different positions on the human body 12 in order to measure the pulse transit time and to detect trends in the blood pressure from the pulse arrival time.

The pulse arrival time is usually determined as a time frame from a maximum peak R of the electrocardiogram to a certain point in time of the photo-plethysmogram. The pulse arrival time may be detected as a time frame from the maximum value R of the electrocardiogram to a minimum value F of the photo-plethysmogram as a foot pulse arrival time PAT_{foot} or to a maximum value T of the photo-plethysmogram as a top pulse arrival time PATₜₒₚ or as a time to the maximum slope of the photo-plethysmogram between the maximum and the minimum value of the photo-plethysmogram. Generally, the pulse arrival time (PAT) is the sum of the pre-ejection period (PEP), determined by a measure of the aortic closure and pulse transit time (PTT), as e.g. described in X. Aubert, J. Muehlsteff, "Non-Invasive Cuff-less Measurements of the Arterial Blood Pressure: What does Pulse-Transit-Time tell us all about?", Proc. ESGCO'06, Jena, Germany, May 2006 and J. Muehlsteff, X. Aubert, M. Schuett, "Cuff-less Estimation of Systolic Blood Pressure for Short Effort Bicycle Tests: The Prominent Role of the Pre-Ejection Period", EMBC'06, pp. xy, New York, 2006.

PTT may be used as a marker of blood pressure changes due its well-defined relation to blood pressure and is based on pulse propagation in elastic arteries. However, PEP - the period of iso-volumetric contraction - can vary independent of the blood pressure. Therefore, some shortcomings of this technique have been shown for absolute blood pressure tracking, but it provides sufficient performance for tracking blood pressure variations.

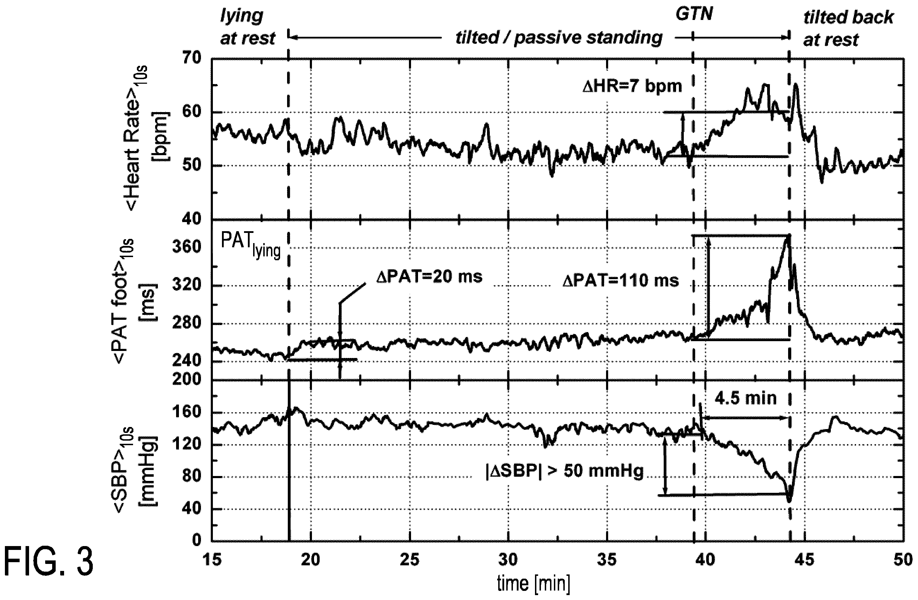

Fig. 3 shows diagram of a heart rate, the parameter PAT_{foot} and the systolic blood pressure (SBP) over time. The diagrams show a typical PAT increase (middle diagram) because of a critical BP decrease (lower diagram). This measurement has been obtained during a tilt table test used as standard procedure to diagnose root causes of syncope. Further studies using a diagnostic passive standing exercise (head-up tilt table testing [HUTT]) showed that a 20 mmHg decrease is associated with a 20 ... 40 ms change in PAT, which can be easily detected, and thus proved the feasibility of using relative PAT changes for early detection of impending faints.

The monitoring system and method according to the present invention achieve an improved reliability of separate measurements using contactless methods and provide access to hemodynamic status and status changes with contactless sensing methods only. They make early detection of critical hemodynamic events of neonates using contactless methods only possible. Further, the invention makes an extension of accessible parameters of current contactless methods such as heart rate, heart rate variability, respiration movements by hemodynamic surrogates not being accessible so far possible and ensures an increased patient safety due to better suited parameter for crisis detection with contactless sensors only. The algorithm to fuse signals enables it to more reliably extract the moment in time when a blood pulse begins. The invention fits well to the current the work flow without extra effort e.g. for placement of additional sensors.

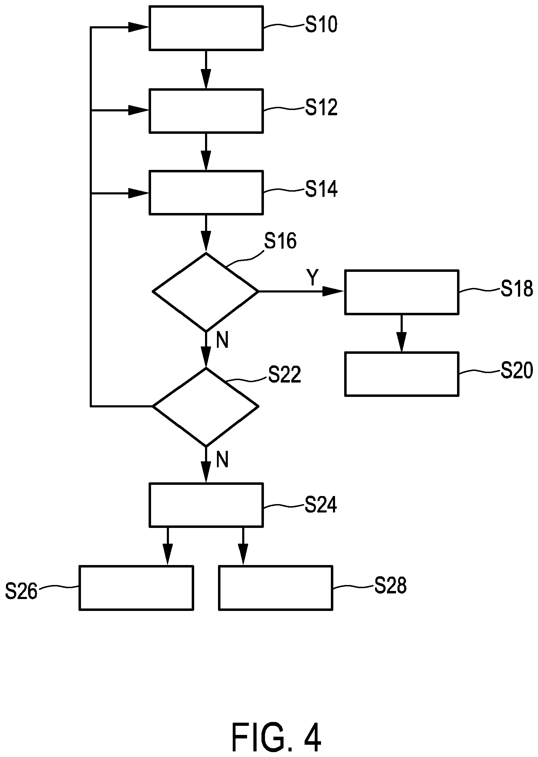

Fig. 4 shows a flow chart illustrating a first embodiment of a monitoring method according to the present invention relating to a plausibility check, improved coverage and reliability of heart rate and respiration rate measurements. In step S10 a measurement of a photo-plethysmogram from the forehead, finger or other body locations is made by analysis of signal strength variations (illuminations) using e.g. a single color or monochrome camera. The region of interest can be detected automatically or defined manually to detect the arrival of a blood pressure wave. In step S12 measurements of capacitive ECG and/or pressure changes in a mattress or textile (snuggle) from which heart rate, heart rate changes and/or respiration rate are detected. In step S14 rates and/or rate changes from all signal sources are compared. In step S16 the reliability and consistency of these measurements is checked. If they are consistent, the measured signals are classified as "good" (S18) and the inferred heart rate and/or respiration rate are extracted from "good" signal only (S20). If they are not consistent, e.g. if one signal is not consistent compared to the other two signals, a check for artifacts in signals is made (S22). If no artifacts are found in at least one signal, a label "highest trust" is assigned to this signal (S24), the inferred heart rate and/or respiration rate are extracted from the "highest trust" signal only (S26) or an average of both signals with higher trust than the remaining one is reported (S28). If artifacts are found the method returns to one of the earlier steps S10, S12, S14.

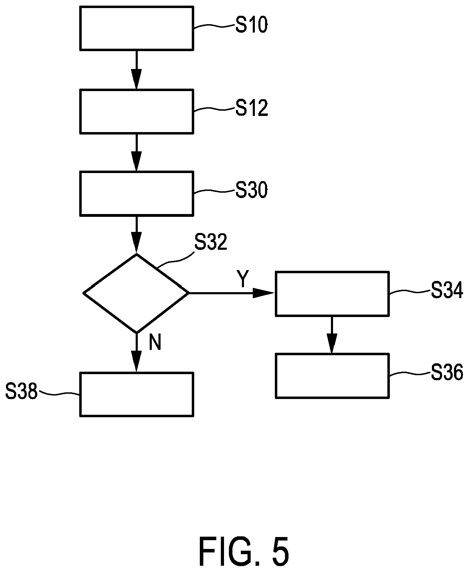

Fig. 5 shows a flow chart illustrating a second embodiment of a monitoring method according to the present invention related to a plausibility check of PAT measures from inference of hemodynamic changes based on the PAT measurement approach. Some of the steps of this method are identical to steps of the method as shown in Fig. 4 and are thus assigned with the same reference sign. After a measurement of a photo-plethysmogram (PPG) in step S10 and measurements of capacitive ECG (cECG) and/or pressure changes in step S12 to infer the starting point of a pressure wave, one or more PAT measures of several or all possible combinations (cECG - PPG, pressure - PPG, cECG - pressure) are extracted in step S30. In step S32 the consistency of the extracted PAT measures and/or PAT changes is checked. If they are consistent the extracted PAT measures and/or PAT changes are reported (S34) and an information on the hemodynamic status of the subject can be derived (S36) based on the above mentioned relationship between PAT measures and hemodynamics. If significant changes on the extracted PAT measures and/or PAT changes are detected a notification or alarm of low signal quality is generated (S38).

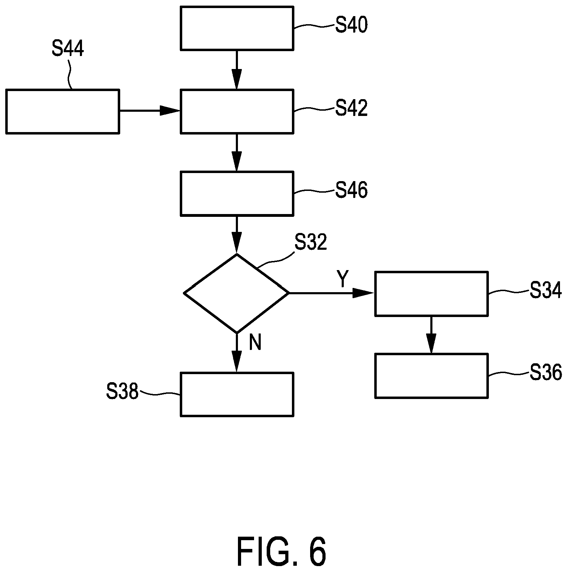

Fig. 6 shows a flow chart illustrating a third embodiment of a monitoring method according to the present invention related to an early detection of hemodynamic changes based on PAT measures compared to reference measurements. In a calibration phase PAT measures are extracted in step S40 as described above with reference to Fig. 5 (steps S10, S12, S30). The PAT measures extracted during this period are then defined as PAT reference (PATref) in step S42. Optionally, the blood pressure is measured (S44) during this measurement, e.g. by use of a cuff-based blood pressure monitor (30 in Fig. 1 providing blood pressure measurement signals 31 used for verifying the result of the evaluation in the evaluation unit 18) for intermittent BP measurements.

In a monitoring phase the PAT measures are extracted in step S46 as described above with reference to Fig. 5 (steps S10, S12, S30) which is then followed by steps S32 to S38 as shown in Fig. 5. Also in the monitoring phase a cuff-based blood pressure measurement (not shown) can be used to calibrate the measurement of PAT measures order to improve the measurement in general.

Access to hemodynamic measures such as blood pressure enables a better assessment of the health status of the patient. The combined analysis of heart rate and blood pressure changes gives insights in regulation mechanisms in the body being normal or not. This is known as baro-reflex response and is important to assess the risk of a patient for a faint as e.g. described in J. Muehlsteff, Pattern Analysis of Pulse Arrival Time and Heart Rate towards Continuous Hemodynamic Monitoring in Low Acuity Settings, BMT 2010, Rostock. Accordingly, in an embodiment the evaluation unit is configured to analyze a heart rate signal related to the heart rate of the subject and blood pressure changes, wherein said heart rate signal is obtained from said sensor signal and/or said PPG signal.

Another application deals with the fact that PAT measures are sensitive to intra-thoracic pressure changes, which occur e.g. during apnea events. The combined interpretation of respiration effort and its impact on PAT measures allow the detection of obstructive events, where typically respiration effort signals show almost normal respiration movements, whereas the intra-thoracic pressure will change abnormal and detect these events with higher sensitivity and specificity compared to measurements using the respiration effort signal only. Accordingly, in an embodiment the sensor unit is configured to acquire a respiration signal related to the respiration rate of the subject and the evaluation unit is configured to extract one or more PAT measures and to analyze the correlation of said respiration signal and one or more of said PAT measures. For this purpose a monitoring system for monitoring a subject may be used comprising an imaging unit configured to obtain a plurality of images of the subject over time, a sensor unit configured to acquire a respiration signal related to the respiration rate of the subject, a PPG unit configured to generate a photo-plethysmography, PPG, signal from said plurality of images, and an evaluation unit configured to extract one or more pulse arrival time, PAT, measures and to analyze the correlation of said respiration signal and one or more of said PAT measures.

Still another application deals with detection and diagnostic of pulsus paradoxus, where an abnormal blood pressure change is observed (typically larger than 10 mmHg) during inspiration. Accordingly, in an embodiment the evaluation unit is configured to determine the presence of an abnormal blood pressure change during inspiration.

The present invention can advantageously be applied in neonatal intensive care, e.g. of a neonate that is located in an incubator or under a radiant warmer or even while being snuggled. The present invention can further be applied in emergency care of patients, in In-Car monitoring, in Home Care and in fitness applications.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A monitoring system (10) for monitoring the hemodynamic status of a subject (100), said monitoring system comprising:
- an imaging unit (12) configured to obtain a plurality of images of the subject (100) from a remote distance over time,
- a sensor unit (14) configured to acquire a sensor signal of the subject (100) related to a vital sign of the subject (100),
- a PPG unit (16) configured to generate photo-plethysmography, PPG, signals from various body sites of the subject from said plurality of images, and
- an evaluation unit (18) configured
to commonly evaluate said sensor signal and said PPG signals,
to extract pulse arrival time, PAT, measures from said sensor signal and said PPG signals, and
to extract a hemodynamic information about the hemodynamic status of the subject (100) from said PAT measures.

2. The monitoring system (10) as claimed in claim 1,
wherein said evaluation unit (18) is configured to determine the change in the blood pressure from the common evaluation of said sensor signal and said PPG signals.

3. The monitoring system (10) as claimed in claim 1,
wherein said evaluation unit (18) is configured to extract as PAT measures the PAT foot, PAT 20%, PAT50 %, PAT80 %, the PAT top, the pulse transit time and/or the pre-ejection period.

4. The monitoring system (10) as claimed in claim 1,
wherein said evaluation unit (18) is configured to determine a start signal of a PAT measure from the sensor signal and an end signal of the PAT measure from a systolic measurement value of the PPG signals.

5. The monitoring system (10) as claimed in claim 1,
wherein said evaluation unit (18) is configured to detect changes in one or more PAT measures and to issue an indication signal indicating if changes in one or more PAT measures have been detected.

6. The monitoring system (10) as claimed in claim 5,
wherein said evaluation unit (18) is configured to compare one or more extracted PAT measures to reference PAT measures previously acquired for the same subject (100) or for a different subject or formed from PAT measures acquired for a plurality of subjects.

7. The monitoring system as claimed in claim 1,
wherein said sensor unit (14) comprises a pressure sensor (141) configured to acquire a pressure sensor signal representing pressure changes allowing the extraction of a heart rate signal related to the heart rate of the subject (100) and/or a respiration signal related to the respiration rate of the subject (100).

8. The monitoring system as claimed in claim 1,
wherein said sensor unit (14) comprises an ECG sensor (142), in particular a capacitive ECG sensor, configured to acquire an ECG sensor signal allowing the extraction of a heart rate signal related to the heart rate of the subject (100).

9. The monitoring system as claimed in claims 7 and 8,
wherein said evaluation unit (18) is configured to extract pulse arrival time, PAT, measures from different combinations of PPG signal, ECG sensor signal and pressure sensor signal, to check said PAT measures for consistency and to use the result of said check in the extraction of a hemodynamic information about the hemodynamic status of the subject (100).

10. The monitoring system as claimed in claim 1,
further comprising a blood pressure measurement unit (30) for measuring the blood pressure of the subject (100).

11. A monitoring method for monitoring the hemodynamic status of a subject (100), said monitoring method comprising:
- obtaining a plurality of images of the subject (100) from a remote distance over time,
- acquiring a sensor signal of the subject (100) related to a vital sign of the subject (100),
- generating photo-plethysmography, PPG, signals from various body sites of the subject from said plurality of images,
- commonly evaluating said sensor signal and said PPG signals,
- extracting pulse arrival time, PAT, measures from said sensor signal and said PPG signals, and
- extracting a hemodynamic information about the hemodynamic status of the subject (100) from said PAT measures.

## Patentansprüche

1. Überwachungssystem (10) zur Überwachung des hämodynamischen Zustands einer Person (100), wobei das genannte Überwachungssystem Folgendes umfasst:
- eine Bildgebungseinheit (12), die konfiguriert ist, um eine Vielzahl von Bildern der Person (100) aus einem entfernten Abstand über die Zeit zu erlangen,
- eine Sensoreinheit (14), die konfiguriert ist, um ein Sensorsignal der Person (100) in Bezug auf einen Vitalparameter der Person (100) zu erfassen,
- eine PPG-Einheit (16), die konfiguriert ist, um Photo-Plethysmographie-Signale (PPG-Signale) von verschiedenen Körperstellen der Person anhand der genannten Vielzahl von Bildern zu erzeugen, und
- eine Evaluierungseinheit (18), die konfiguriert ist
zum gemeinsamen Evaluieren des genannten Sensorsignals und der genannten PPG-Signale,
zum Extrahieren von Pulsankunftszeit- (pulse arrival time, PAT) Messungen aus dem genannten Sensorsignal und den genannten PPG-Signalen, und
zum Extrahieren einer hämodynamischen Information über den hämodynamischen Zustand der Person (100) aus den genannten PAT-Messungen.

2. Überwachungssystem (10) nach Anspruch 1,
wobei die genannte Evaluierungseinheit (18) konfiguriert ist, um die Änderung im Blutdruck anhand der gemeinsamen Evaluierung des genannten Sensorsignals und der genannten PPG-Signale zu ermitteln.

3. Überwachungssystem (10) nach Anspruch 1,
wobei die genannte Evaluierungseinheit (18) konfiguriert ist, um als PAT-Messungen die PAT Fuß, PAT 20%, PAT 50%, PAT 80%, die PAT oben, die Pulslaufzeit und/oder die Pre-Ejektionsperiode zu extrahieren.

4. Überwachungssystem (10) nach Anspruch 1,
wobei die genannte Evaluierungseinheit (18) konfiguriert ist, um ein Startsignal einer PAT-Messung anhand des Sensorsignals und ein Endsignal der PAT-Messung anhand eines systolischen Messwerts der PPG-Signale zu ermitteln.

5. Überwachungssystem (10) nach Anspruch 1,
wobei die genannte Überwachungseinheit (18) konfiguriert ist, um Änderungen in einer oder mehreren PAT-Messungen zu detektieren und ein Hinweissignal auszugeben, ob Änderungen in einer oder mehreren PAT-Messungen detektiert worden sind.

6. Überwachungssystem (10) nach Anspruch 5,
wobei die genannte Evaluierungseinheit (18) konfiguriert ist, um eine oder mehrere extrahierte PAT-Messungen mit Referenz-PAT-Messungen zu vergleichen, die zuvor für die gleiche Person (100) oder für eine andere Person erfasst wurden oder aus PAT-Messungen gebildet werden, die für eine Vielzahl von Personen erfasst wurden.

7. Überwachungssystem nach Anspruch 1,
wobei die genannte Sensoreinheit (14) einen Drucksensor (141) umfasst, der konfiguriert ist, um ein Druckänderungen darstellendes Drucksensorsignal zu erfassen, das das Extrahieren eines Herzfrequenzsignals in Bezug auf die Herzfrequenz der Person (100) und/oder eines Atemsignals in Bezug auf die Atemfrequenz der Person (100) erlaubt.

8. Überwachungssystem nach Anspruch 1,
wobei die genannte Sensoreinheit (14) einen EKG-Sensor (142), insbesondere einen kapazitiven EKG-Sensor, umfasst, der konfiguriert ist, um ein EKG-Sensorsignal zu erfassen, das das Extrahieren eines Herzfrequenzsignals in Bezug auf die Herzfrequenz der Person (100) erlaubt.

9. Überwachungssystem nach den Ansprüchen 7 und 8,
wobei die genannte Evaluierungseinheit (18) konfiguriert ist, um Pulsankunftszeit-Messungen (PAT-Messungen) aus verschiedenen Kombinationen von PPG-Signal, EKG-Sensorsignal und Drucksensorsignal zu extrahieren, um die genannten PAT-Messungen auf Konsistenz zu prüfen und um das Ergebnis der genannten Prüfung bei der Extraktion von hämodynamischen Informationen über den hämodynamischen Zustand der Person (100) zu verwenden.

10. Überwachungssystem nach Anspruch 1,
weiterhin umfassend eine Blutdruckmesseinheit (30) zum Messen des Blutdrucks der Person (100).

11. Überwachungsverfahren zur Überwachung des hämodynamischen Zustands einer Person (100), wobei das genannte Überwachungsverfahren Folgendes umfasst:
- Erlangen einer Vielzahl von Bildern der Person (100) aus einem entfernten Abstand über die Zeit,
- Erfassen eines Sensorsignals der Person (100) in Bezug auf einen Vitalparameter der Person (100),
- Erzeugen von Photo-Plethysmographie-Signalen (PPG-Signalen) von verschiedenen Körperstellen der Person anhand der genannten Vielzahl von Bildern,
- gemeinsames Evaluieren des genannten Sensorsignals und der genannten PPG-Signale,
- Extrahieren der Pulsankunftszeit- (pulse arrival time, PAT) Messungen aus dem genannten Sensorsignal und den genannten PPG-Signalen, und
- Extrahieren einer hämodynamischen Information über den hämodynamischen Zustand der Person (100) aus den genannten PAT-Messungen.

## Revendications

1. Système de surveillance (10) pour surveiller l'état hémodynamique d'un sujet (100), ledit système de surveillance comprenant :
- une unité d'imagerie (12) configurée pour obtenir une pluralité d'images du sujet (100) à distance, au fil du temps,
- une unité de capteur (14) configurée pour acquérir un signal de capteur du sujet (100) relativement à un signe vital du sujet (100),
- une unité PPG (16) configurée pour générer des signaux de photo-pléthysmographie, une PPG, provenant de différents endroits du corps du sujet à partir de ladite pluralité d'images, et
- une unité d'évaluation (18) configurée
pour évaluer communément ledit signal de capteur et lesdits signaux PPG,
pour extraire le temps d'arrivée de l'impulsion, le PAT, des mesures provenant dudit signal de capteur et desdits signaux PPG et
pour extraire une information hémodynamique concernant l'état hémodynamique du sujet (100) desdites mesures PAT.

2. Système de surveillance (10) selon la revendication 1,
dans lequel ladite unité d'évaluation (18) est configurée pour déterminer le changement de pression artérielle, à partir de l'évaluation commune dudit signal de capteur et desdits signaux PPG.

3. Système de surveillance (100) selon la revendication 1,
dans lequel ladite unité d'évaluation (18) est configurée pour extraire comme mesures de PAT, le PAT au pied, le PAT à 20 %, le PAT à 50 %, le PAT à 80 %, le PAT au niveau supérieur, le temps de transit d'impulsion et/ou la période de pré-éjection.

4. Système de surveillance (10) selon la revendication 1,
dans lequel ladite unité d'évaluation (18) est configurée pour déterminer un signal de départ d'une mesure PAT à partir du signal de capteur et un signal de fin de la mesure de PAT à partir d'une valeur de mesure systolique des signaux PPG.

5. Système de surveillance (10) selon la revendication 1,
dans lequel ladite unité d'évaluation (18) est configurée pour détecter des changements d'une ou plusieurs mesures PAT et pour émettre un signal d'indication indiquant si des changements d'une ou plusieurs mesures PAT ont été détectés.

6. Système de surveillance (10) selon la revendication 5,
dans lequel ladite unité d'évaluation (18) est configurée pour comparer une ou plusieurs mesures PAT extraites à des mesures PAT de référence précédemment acquises pour le même sujet (100) ou pour un sujet différent ou constituées à partir de mesures PAT acquises pour une pluralité de sujets.

7. Système de surveillance selon la revendication 1,
dans lequel ladite unité de capteur (14) comprend un capteur de pression (141) configuré pour acquérir un signal de capteur de pression représentant des changements de pression permettant l'extraction d'un signal de fréquence cardiaque relativement à la fréquence cardiaque du sujet (100) et/ou un signal de respiration relatif au rythme respiratoire du sujet (100).

8. Système de surveillance selon la revendication 1,
dans lequel ladite unité de capteur (14) comprend un capteur ECG (142), en particulier un capteur ECG capacitif, configuré pour acquérir un signal de capteur ECG permettant l'extraction d'un signal de fréquence cardiaque relativement à la fréquence cardiaque du sujet (100).

9. Système de surveillance selon les revendications 7 et 8,
dans lequel ladite unité d'évaluation (18) est configurée pour extraire un temps d'arrivée de l'impulsion, PAT, des mesures provenant de différentes combinaisons de signal PPG, de signal de capteur ECG et de signal de capteur de pression, afin de vérifier la cohérence desdites mesures PAT et d'utiliser le résultat dudit contrôle dans l'extraction d'une information hémodynamique concernant l'état hémodynamique du sujet (100).

10. Système de surveillance selon la revendication 1,
comprenant en outre une unité de mesure de la pression artérielle (30) pour mesurer la pression artérielle du sujet (100).

11. Procédé de surveillance pour surveiller l'état hémodynamique d'un sujet (100), ledit procédé de surveillance comprenant :
- l'obtention d'une pluralité d'images du sujet (100) à distance au fil du temps,
- l'acquisition d'un signal de capteur du sujet (100) relativement à un signe vital dudit sujet (100)
- la génération de signaux d'une photo-pléthysmographie, PPG, provenant de différents endroits du corps du sujet à partir de ladite pluralité d'images,
- l'évaluation commune dudit signal de capteur et desdits signaux PPG
- l'extraction du temps d'arrivée d'impulsion, PAT, des mesures provenant dudit signal de capteur et desdits signaux PPG et
- l'extraction d'informations hémodynamiques concernant l'état hémodynamique du sujet (100) à partir desdites mesures PAT.
